(19) **Europäisches Patentamt**

**European Patent Office**

**Office européen des brevets**

(11) **EP 1 365 027 A2**

(12) **EUROPEAN PATENT APPLICATION**
published in accordance with Art. 158(3) EPC

(43) Date of publication:
**26.11.2003 Bulletin 2003/48**

(51) Int Cl.⁷: **C12N 15/62**, C12N 5/10,
C07K 14/47, C07K 19/00

(21) Application number: **01934373.0**

(22) Date of filing: **28.05.2001**

(86) International application number:
**PCT/JP01/04456**

(87) International publication number:
**WO 01/090382 (29.11.2001 Gazette 2001/48)**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **26.05.2000 JP 2000156209**

(71) Applicant: **MOCHIDA PHARMACEUTICAL CO.,
LTD.**
**Shinjuku-ku Tokyo 160-8515 (JP)**

(72) Inventor: **TOUMA, Jyunko**
**c/o MOCHIDA PHARMACEUTICAL CO., LTD.**
**Tokyo 160-8515 (JP)**

(74) Representative: **Grünecker, Kinkeldey,
Stockmair & Schwanhäusser Anwaltssozietät
Maximilianstrasse 58
80538 München (DE)**

(54) **FAS LIGAND-FUSED PROTEINS**

(57)   Provided by this invention is a fusion protein which is capable of binding to Fas, and which comprises a peptide comprising at least a part of the amino acid sequence of Fas ligand, a peptide having oligomerization ability, and a peptide which increases recombinant protein production. Also provided are a method for using the FLAG-like peptide for the purpose of increasing the production of the recombinant protein, and a method for using the FLAG-like peptide for the purpose of increasing the biological activity of the fusion protein of the leucine zipper and the transmembrane protein.

**Description**

TECHNICAL FIELD

This invention provides a fusion protein which has a biological activity of Fas ligand; a DNA coding for such fusion protein; an expression vector including such DNA; and a transformant transformed by such vector. This invention also provides a method for increasing the production of a recombinant protein which is useful in producing such fusion protein and which is simultaneously a universal method applicable to any type of recombinant protein; and a method for increasing the biological activity of the fusion protein of leucine zipper and a transmembrane protein.

BACKGROUND TECHNOLOGY

Fas is a type I transmembrane protein with a molecular weight of about 45 kD which belongs to TNF receptor family, and Fas is recognized by anti-Fas antibody (Yonehara et al., J.Exp.Med. 169: 1747, 1989) or APO-1 antibody (Trauth et al., Science 267: 1456, 1989). Fas transduces apoptotic signal to the cell as a cell surface antigen.

Human Fas ligand is a type II transmembrane protein with a molecular weight of about 40 kD which belongs to TNF family, and this human Fas ligand was first reported by the group of Nagata et al. as a biological molecule which induces apoptosis to the Fas-expressing cell. (Takahashi et al., International Immunology 6: 1567, 1994). As in the case of TNF, human Fas ligand is believed to form a trimer in the body (Tanaka et al., EMBO J. 14: 1129, 1995). Human Fas ligand is also highly homologous to rat Fas ligand (Suda et al., Cell 75: 1169, 1993) and mouse Fas ligand (Takahashi et al., Cell 76: 969, 1994) in its extracellular domain, and human Fas ligand is capable of recognizing not only the human Fas but also the mouse Fas, and induces apoptosis. Similarly, rat and mouse Fas ligands are capable of recognizing the human Fas and inducing apoptosis.

In the meanwhile, investigation on Fas-mediated intracellular transduction of the apoptotic signal has proceeded. There has been reported identification and cloning of a factor which transduces or suppresses the signal by interacting with the intracellular domain, and in particular, the domain called "death domain" of the Fas. Also reported are involvement of a series of cysteine proteases called "Caspase" in the Fas-mediated transduction of the apoptotic signal.

Recent studies also indicate involvement of the apoptosis, and in particular, the Fas-mediated apoptosis in various diseases and physiological conditions. For example, there has been indicated possibilities of the involvement of abnormality of the Fas-mediated apoptosis in certain autoimmune diseases and hepatocyte death in viral fulminant hepatitis. Also indicated are possibility of the Fas-Fas ligand system being involved in functions other than the apoptosis, for example, inflammatory action by acting on neutrophil (Kayagaki et al., Clinical Immunology 28: 667, 1996).

A significant challenge in the recombinant protein technology has often been the expression of a biologically active recombinant protein having adequate tertiary and quaternary structures. The most important of such challenge has been expression of a transmembrane protein in the form of a soluble protein with its biological activity retained since a soluble protein is useful in the production of drugs wherein a highly purified protein is required in a large amount.

Production of a transmembrane protein in soluble form has been accomplished by removing the transmembrane domain and the cytoplasmic domain, and adding an adequate signal peptide to thereby enable secretion of the desired protein in soluble form (Smith et al., Science 238: 1704, 1987; Treiger et al., J. Immunol. 136: 4099, 1986). In the case of the Fas ligand, there has been disclosed a fusion protein of the extracellular domain of human Fas ligand with the signal sequence and the extracellular domain of CD8 (USP 6,001,962). However, the soluble Fas ligand has been known to exhibit a biological activity which is inferior to the full length Fas ligand, and no Fas ligand having the activity of the level that would enable its use in the field of medicine has so far been provided. In the case of CD40 ligand which is a member of the TNF family and whose activity is believed to be highly dependent on the formation of a trimer, a method for producing a trimer CD40 ligand by using leucine zipper has been disclosed (USP 5,716,805). However, production of a fusion protein including a peptide having oligomerization ability such as leucine zipper often resulted in the significant reduction in the yield of the protein in soluble form with its function retained, and production of such protein at a sufficient amount has often been difficult (Pack et al., J.Mol.Biol. 246: 28, 1995).

FLAG-like peptide provides an epitope which does not alter the biological activity of the resulting fusion protein and which is recognized by the specific monoclonal antibody, and this enables rapid detection as well as convenient purification of the expressed fusion protein. Until recently, FLAG-like peptide has been used merely as a tag for purification. It was then reported that, when the membrane-binding domain of a membrane-bound protein is prepared in the soluble form by binding the FLAG peptide to the membrane-binding domain and thereafter removing the FLAG peptide by using enterokinase, the membrane-binding domain recovers its membrane-binding activity (Chen et al., Biochemistry 37: 13643, 1998). However, its has been unknown that the FLAG-like peptide has the effect of increasing the production of the recombinant protein, and also, the effect of increasing the biological activity the fusion protein of the leucine zipper and the transmembrane protein.

An object of the present invention is to provide a Fas ligand fusion protein having a high biological activity in a large amount and in a convenient manner. Also provided are methods which can be used for the production of such Fas ligand fusion protein, namely, a convenient method capable of increasing the production of the recombinant protein, and a method for increasing the biological activity of the fusion protein of leucine zipper and a transmembrane protein. Production of the Fas ligand fusion protein exhibiting a high biological activity in a large amount by a simple procedure should facilitate development of therapeutic agents for diseases associated with the Fas-mediated apoptosis. Such production of the Fas ligand fusion protein is also critical in elucidating intracellular signal transduction pathway induced by the Fas-Fas ligand binding and in searching novel factors which are involved in the regulation of the Fas-Fas ligand interaction, and therefore, highly demanded in the medical and many other fields.

In order to provide the Fas ligand which is useful in the field of medicine, it is important that the Fas ligand has reliable, high biological activity, and that the Fas ligand can be produced in a large amount. No production of such Fas ligand has so far been proposed.

DISCLOSURE OF THE INVENTION

The inventor of the present invention has continued extensive investigation to produce the Fas ligand exhibiting a high biological activity in a large amount, and as a result of such investigation, the inventor succeeded in producing the Fas ligand of high activity in a large amount by producing the Fas ligand in the form of a fusion protein with leucine zipper and FLAG-like peptide. This invention discloses that the FLAG-like peptide has the effect of increasing the production of the recombinant protein, as well as the effect of increasing the biological activity of the fusion protein of the leucine zipper and the transmembrane protein.

According to the first aspect of the present invention, there is provided a fusion protein which is capable of binding to Fas, and which comprises a peptide comprising at least a part of the amino acid sequence of Fas ligand, a peptide having oligomerization ability, and a peptide which increases recombinant protein production. The second aspect of the present invention is a DNA having the nucleotide sequence coding for the fusion protein according to the first aspect of the present invention. The third aspect of the present invention is an expression vector containing the DNA according to the second aspect of the present invention. The fourth aspect of the present invention is a transformant transformed by the expression vector according to the third aspect of the present invention.

The fifth aspect of the present invention is a method for increasing the production of the recombinant protein, which is characterized by that the desired protein is produced as a fusion protein with FLAG-like peptide. The sixth aspect of the present invention is a method for producing a recombinant protein comprising the steps of producing an expression vector including DNA fragment of the nucleotide sequence coding for the desired protein ligated to the nucleotide sequence coding for FLAG-like peptide with the reading frame matched; introducing said expression vector in a host such as an appropriate cell or a micro-organism; cultivating the resulting transformant in the condition suitable for expression; and recovering the recombinant protein from the culture mixture and purifying the recovered recombinant protein to thereby increase production of said desired protein. The seventh aspect of the present invention is a method for increasing the biological activity of a fusion protein of leucine zipper and a transmembrane protein wherein FLAG-like peptide is further ligated to the fusion protein in the process of producing said fusion protein.

BRIEF DESCRIPTION OF THE DRAWINGS

FIG. 1 is a view showing apoptosis-inducing activity of various Fas ligand fusion proteins for Jurkat cell.
FIG. 2 is a view schematically showing primary structure of various human Fas ligand fusion proteins.
FIG. 3 is a view showing the results of silver staining of purified FLAG - IleZip - shFasL after electrophoresis.

PREFERRED EMBODIMENT OF THE INVENTION

The present invention is hereinafter described in detail.

First, the fusion protein according to the first aspect of the present invention is described. The term "Fas ligand" used herein designates a substance which at least has the biological activity of binding to Fas, and more preferably, a substance which has the activity to induce apoptosis of the Fas-expressing cell. To be more specific, the term "biological activity" used in relation to the Fas ligand is the Fas-binding activity, and more preferably, the activity of inducing apoptosis to the Fas-expressing cell. Apoptosis is believed to be induced by the Fas ligand through binding of the Fas ligand to the Fas on the cell surface and the subsequent Fas-mediated transduction of the apoptotic signal to the cell. The Fas and the Fas ligand in this context are not limited for their source, and they may be the one derived from any of the animals including human, mouse, rat, guinea pig, chicken, rabbit, pig, sheep, cow, horse, monkey, cat, dog, and marmot. The Fas and the Fas ligand, however, are preferably those of human origin.

The "peptide (a) comprising at least a part of the amino acid sequence of Fas ligand" according to the first aspect of the present invention is preferably a peptide which has the Fas ligand biological activity which either comprises all of the amino acid sequence of SEQ ID NO:1 of the Sequence Listing or arbitrary part of arbitrary length in said amino acid sequence. However, as long as the peptide retains its Fas ligand biological activity, the peptide (a) may be a peptide which is defined by the amino acid sequence comprising an arbitrary part in the above-described amino acid sequence and at least one amino acid of any type such as Met attached to either or both of the N and C terminals of said part; or a peptide which comprises an amino acid sequence wherein one to several amino acids have been mutated, deleted, substituted, or added in the above-described amino acid sequence. For example, the amino acid sequence of the mouse or rat Fas ligand may be described as an amino acid sequence wherein substitution and deletion had occurred at a plurality of positions in the human Fas ligand, and both the rat and the mouse Fas ligands are known to have the Fas ligand biological activity. Similarly, the Fas ligand from rhesus monkey (Wang et al., Human Immunology 59: 599, 1998), guinea pig, chicken, rabbit, pig, sheep, cow, horse, monkey, cat, dog, marmot, and other animals are within the scope of the peptide comprising at least a part of the amino acid sequence of Fas ligand according to the first aspect of the present invention as long as the peptide has the Fas ligand biological activity. The most preferable example of the peptide comprising at least a part of the amino acid sequence of Fas ligand is the extracellular domain of Fas ligand. The extracellular domain of Fas ligand corresponds to amino acids 103 to 281 in the human Fas ligand shown in SEQ ID NO:1 of the Sequence Listing. Another preferable example is amino acids 130 to 281 which corresponds to the soluble human Fas ligand which is cleaved from the membrane-type human Fas ligand in the living body by a protease. The fusion protein according to the first aspect of the present invention may be either the one with or without the apoptosis-inducing activity as long as it has the Fas-binding activity. When the fusion protein is the one which binds to the Fas but which does not induce the apoptosis, such fusion protein can be used as a substance that competes with the Fas ligand in the living body, and such fusion protein can be used for artificial suppression of the apoptosis. The fusion protein according to the first aspect of the present invention, however, is preferably the one having the apoptosis-inducing activity. In the case of the human Fas ligand, amino acids 145 to 281 from the N terminal are known to be critical for the apoptosis-inducing activity. In view of the situation as described above, the fusion protein according to the first aspect of the present invention is preferably the one comprising the amino acids 145 to 281 of the human Fas ligand as at least a part of the amino acid sequence of the Fas ligand. In order to obtain a fusion protein with a stronger apoptosis-inducing activity, however, it is more preferable that the fusion protein comprising the amino acid sequence corresponding to the sequence of amino acid 144 and later in the human Fas ligand.

(b) The "peptide having oligomerization ability" is the peptide which has the ability of self-associating into a dimer, a trimer, or a higher oligomer, and in most cases, such peptide is capable of forming α-helix, β-sheet, or other secondary structure. A preferable example of such peptide is leucine zipper.

"Leucine zipper" is a term which is used to designate a repetitive 7-residue motif represented by $(abcdefg)_n$ (wherein n is 4 or 5) which exists as a conserved domain in various proteins. In the formula, a and d generally represent a hydrophobic residue such as leucine or isoleucine, and they are arranged on the same surface of the helix (McLachlan et al., J.Mol.Biol. 98: 293, 1975). The leucine residue located at position d contributes for the high hydrophobic stabilization energy, and this is important for dimer formation (Krystek et al., Int.J.Peptide Res. 38: 229, 1991). In addition, substitution of the amino acid residues of the leucine zipper corresponding to the residues a and d in the above formula has been found to result in the change of oligomerization properties (Harbury et al., Science 262: 1401, 1993). When all of the residues at position a are substituted by isoleucine, the leucine zipper still forms a parallel dimer, and when all leucine residues at position d are substituted with isoleucine in addition to the substitution at the position a, the resulting peptide spontaneously forms a trimeric parallel helix in the solution. When all amino acids at position d are substituted with isoleucine, and all amino acids at position a are substituted with leucine, a tetramer is formed. Such substituted peptides are also referred to as a leucine zipper as long as the oligomerization mechanism is the same. The most preferable example of the leucine zipper is the one having the sequence described in SEQ ID NO:2. The leucine zipper is not limited to such sequence as long as it has the oligomerization ability. Other known examples of the peptide having the oligomerization ability include the sequence involved in the tetramerization of p53, platelet factor 4 and the sequence which is a part of histon H3 or H4 protein (These sequences are described in detail in JP 11-508126 A, which is herein cited and incorporated by reference). The peptide wherein mutation, addition, deletion, or substitution of at least one amino acid has taken place at any position in the amino acid sequence are also included within the leucine zipper as long as the peptide has the leucine zipper biological activity.

(c) The "peptide which increases recombinant protein production" is the peptide which, for example, has the effect of increasing the amount of the fusion protein recovered from the supernatant in the production of a fusion protein of a mammal cell, or the effect of increasing the amount of the fusion protein recovered from the E. coli lysate in the production of a fusion protein by using E. coli. The inventor of the present invention found, for the first time, that a typical peptide which increases the production of the recombinant protein is the FLAG-like peptide. In the specification of the invention, FLAG-like peptide is preferably a FLAG peptide comprising 8 amino acids of Asp-Tyr-Lys-Asp-Asp-Asp-Asp-Lys (Hopper et al., Bio/Technology 6: 1204, 1988; US Registered Trademark No. 1557485). However, as long as the peptide has the effects of increasing the production of the recombinant protein and more preferably, has the effect of increasing the biological activity of the fusion protein of leucine zipper and transmembrane protein, the peptide (c) may be a peptide which is defined by the amino acid sequence comprising an arbitrary part of the above-described amino acid sequence and at least one amino acid of any type such as Met attached to either or both of the N and C terminals of said part; or a peptide which comprises an amino acid sequence wherein one to several amino acids have been mutated, deleted, substituted, or added in the above-described amino acid sequence. Such peptide is designated a FLAG-like peptide. For example, a peptide comprising the amino acid sequence of Asp-Leu-Tyr-Asp-Asp-Asp-Asp-Lys may also be used as a peptide having an equivalent function as the peptide comprising the above-described 8 amino acid sequence. In other words, a preferable example of the FALG-like peptide may be represented as a peptide comprising the amino acid sequence of Asp-B-Z-Asp-Asp-Asp-Asp-Lys (wherein B-Z is Tyr-Lys or Leu-Tyr). Another preferable example may be represented as a peptide comprising the amino acid sequence of Asp-Tyr-Lys-$X_{1-n}$-R (wherein R represents Lys, Arg, Met or Asn, and $X_{1-n}$ represents an amino acid other than Lys, Arg, Met and Asn) as described in JP 7-4255 B. In this formula, n is not limited to any particular value, but is preferably 3 to 5.

The fusion protein of the present invention comprises a peptide comprising at least a part of the amino acid sequence of Fas ligand, a peptide having oligomerization ability, and a peptide which increases recombinant protein production, and as long as the fusion protein retains the Fas-binding capability as its nature, and preferably the activity of inducing apoptosis of the Fas-expressing cell, the order how these three peptide are ligated is not limited, and the fusion protein may include any linker sequence or signal sequence. In the case of Fas ligand which is known to interact with the Fas on its C terminal side, the peptide having oligomerization ability and the peptide which increases recombinant protein production are preferably ligated on N terminal side of the peptide comprising at least a part of the amino acid sequence of Fas ligand. The linker sequence are well known in the art. Typical examples of the signal sequence include signal sequences which promote protein secretion such as mouse T lymphocyte antigen CD8 signal sequence, baculovirus gp67 protein signal sequence, G-CSF signal sequence, and human Fas signal sequence.

A preferred embodiment of the fusion protein of the present invention is the fusion protein wherein the FLAG-like peptide, the leucine zipper, and the extracellular domain of human Fas ligand are ligated in this order from the N terminal side. The most preferred is the one having the amino acid sequence of SEQ ID NO:4 of the Sequence Listing, and this is a fusion protein wherein the human Fas signal sequence, the FLAG peptide, the leucine zipper, and the extracellular domain of human Fas ligand are ligated in this order from the N terminal side. Also included within the scope of the fusion protein of the present invention are the peptide defined by the amino acid sequence comprising an arbitrary part of the amino acid sequence of SEQ ID NO:4 and at least one amino acid of any type such as Met attached to either or both of the N and C terminals of said part; or the peptide which comprises an amino acid sequence wherein one to several amino acids have been mutated, deleted, substituted, or added in the above-described amino acid sequence.

The fusion protein of the present invention may be evaluated for its biological activity, namely, for the Fas-binding ability or the apoptosis-inducing activity by the WST-1 assay shown in the Examples and other assays used in the art. WST-1 assay may be conducted by Premix WST-1 Cell Proliferation Assay System (Takara Shuzo Co.). The Fas expressing cell used in this assay is preferably Jurkat cell which is a cell line derived from human T cell. Assays other than the WST-1 assay which may be used include an assay of the Fas-binding activity wherein immunoprecipitation of the Fas with the fusion protein of the present invention is observed, and an flow cytometry assay with an fluorolabeled antibody which can recognize the fusion protein that became bound to the Fas-expressing cell. The method which can be used for assaying the apoptosis-inducing activity include the method of Rouvier (J.Exp.Med. 177: 195, 1993). The fusion protein of the present invention may preferably have an apoptosis-inducing activity such that the cell viability is 50% or less in the WST-1 assay described in the Example when the fusion protein is added at an amount of 3 ng/mL. In this assay, the fusion protein is evaluated to have a higher apoptosis-inducing activity when the cell viability is low. The fusion protein of the present invention having a high apoptosis-inducing activity is quite useful when it is used as a drug since the therapeutically effective dose is reduced and decrease of the side effects as well as the production cost are enabled. In a more preferable embodiment of the present invention, the fusion protein has an apoptosis-inducing activity such that the cell viability is 20% or less upon addition of the fusion protein at an amount of 3 ng/mL in the assay described above.

The fusion protein of the present invention has a characteristic feature that, in this fusion protein with both FLAG-like peptide and leucine Zipper, increase in the activity as the Fas ligand and increase in the production of the fusion protein are simultaneously realized. In the case of a molecule like Fas ligand which can exist as a polymer in a living body, it was within the expectation that production of the Fas ligand in the form of a fusion protein with the leucine zipper should enable production of a recombinant protein wherein the Fas ligand activity is retained. However, it was utterly beyond expectation that a combination of the leucine zipper with the FLAG peptide should result in the increase of the activity as well as in the increase of the amount produced compared to the use of the leucine zipper alone. To be more specific, the fusion protein of the present invention is produced in an amount that is higher by at least 1.5 folds, preferably by at least 2 folds, more preferably by at least 3 folds, still more preferably by at least 20 folds, and most preferably by at least 30 folds when compared to the fusion protein that had been fused only with the leucine zipper, and the produced fusion protein exhibits the Fas ligand biological activity which is higher at least by 1.5 folds, preferably by at least 2 folds, more preferably by at least 3 folds, still more preferably by at least 5 folds, and most preferably by at least 10 folds when compared to the fusion protein fused only with the leucine zipper.

The fusion protein according to the first aspect of the present invention is produced by a genetic engineering means as a recombinant protein. In a typical process of producing the fusion protein by a genetic engineering means, the fusion protein is produced by transforming an adequate host cell by using the novel DNA according to the second aspect of the invention or the expression vector according to the third aspect of the invention as will be described below, and cultivating the resulting transformant to recover the culture mixture from which the target fusion protein is purified. Another typical process of producing the fusion protein by a genetic engineering means is synthesis of the fusion protein in a cell-free system by using the DNA or the recombinant DNA molecule (Sambrook et al., Molecular Cloning 2nd ed., Cold Spring Harbor Laboratory, New York. 1989). The preferable method for producing the fusion protein by a genetic engineering means will be described with regard to the sixth aspect of the present invention.

Next, the DNA according to the second aspect of the present invention is described. This DNA has a nucleotide sequence which codes for the fusion protein according to the first aspect of the present invention. Since the fusion protein comprises a peptide comprising at least a part of the amino acid sequence of Fas ligand, a peptide having oligomerization ability, and a peptide which increases recombinant protein production, the DNA comprises the nucleotide sequences coding for these three types of peptides ligated to each other with the reading frame matched. As long as the fusion protein coded by the DNA retains the Fas-binding capability as its nature, and preferably, the activity of inducing apoptosis to the Fas-expressing cell, the order how the nucleotide sequence coding for these three peptide are ligated is not limited, and the DNA may include the nucleotide sequence coding for any linker sequence or signal sequence. In the case of Fas ligand which is known to interact with the Fas on its C terminal side, the nucleotide sequence coding for the peptide having oligomerization ability and the peptide which increases recombinant protein production are preferably ligated to 5' terminal side of the nucleotide sequence coding for the peptide comprising at least a part of the amino acid sequence of Fas ligand.

The DNAs coding for the polypeptides having the same function are quite often homologous with each other irrespective of the source animal or the source individual, and these DNAs are often hybridizable to each other. Also, polymorphism of an amino acid sequences mostly occurs within the extent such that the DNAs coding for such amino acid sequences remain mutually hybridizable. For example, DNA cloning accomplished by means of hybridization indicates that DNAs coding for different amino acid sequences are mutually hybridizable. The polypeptides having the amino acid sequences coded by the mutually hybridizable DNAs are believed to serve substantially identical function.

In view of the situation as described above, the fusion protein of the present invention may also be characterized as a fusion protein which has Fas-binding activity and which has the amino acid sequence coded by a nucleotide sequence which hybridizes to a nucleotide sequence complementary to the nucleotide sequence coding for the amino acid sequence.

The DNA fragments having the nucleotide sequence coding for each of the three types of peptides are not limited by the way how they have been produced. For example, they may be chemically synthesized fragments, fragments cloned from an adequate DNA library, fragments prepared by cleaving adequate parts from other recombinant DNA by restriction enzyme treatment, or fragments prepared by PCR amplification using other recombinant DNA for the template with adequate primers. These DNA fragment may be ligated by any of known ligation methods, or produced into a continuous DNA fragment by PCR using the fragment mixture for the template with adequate primers.

Next, the expression vector according to the third aspect of the present invention is described. The expression vector according to the third aspect of the present invention contains the DNA according to the second aspect of the present invention, and this DNA fragment has been ligated to an adequate transcriptional and/or translational regulatory nucleotide sequence such as those obtained from a mammal, microorganism, virus, or insect gene in a functional manner. Typical regulatory sequences include sequences playing regulatory role in the gene expression (for example, transcription promoter or enhancer), operator sequence controlling the transcription, sequence coding for the mRNA ribosome binding site, polyadenylation site, splice donor and acceptor sites, and adequate sequence controlling the initiation and termination of the transcription and translation. The need for such nucleotide sequence is determined by the intended use of the expression vector.

The expression vector according to the third aspect of the present invention can be produced by introducing the DNA according to the second aspect of the present invention in an arbitrary vector. If necessary, the DNA can be introduced in the vector with other nucleotide sequence. The method for introducing the DNA in a vector is known in the art (Sambrook et al., Molecular Cloning 2nd ed., Cold Spring Harbor Laboratory, New York, 1989). To be more specific, the DNA and the vector may be respectively digested with adequate restriction enzymes, and the resulting fragments of the DNA and the vector may be ligated using a DNA ligase. The vector may be any vector selected from a plasmid vector, phage vector, virus vector, and the like, and to be more specific, an adequate vector may be selected from pSV2-dhfr, pBluescriptII, pPIC9K, λZapII, λgt11, pEF-BOS, and the like. The expression vector may preferably contain Escherichia coli replication origin, marker gene, and polyadenylation sequence in addition to the DNA according to the second aspect of the present invention. Also preferred as the expression vector of the invention are those further containing trp promoter or lac promoter functioning in Escherichia coli, the promoter for alcohol oxidase (AOX) 1 functioning in yeast, polyhedron promoter functioning in an insect cell, the promoter for SV40, the promoter for SRα, or the promoter for human elongation factor 1α (EF1α) functioning in an animal cell.

Next, the transformant according to the fourth aspect of the present invention is described. The transformant according to the fourth aspect of the present invention is the one that has been transformed by the expression vector according to the third aspect of the present invention. To be more specific, the transformant according to the fourth aspect of the present invention is the one that has been transformed by directly introducing the expression vector according to the third aspect of the present invention in an adequate host cell or microorganism. The method that can be employed in introducing the expression vector according to the third aspect of the present invention in the host cell include electroporation, protoplast fusion, alkaline metal process, calcium phosphate precipitation, DEAE dextran process, microinjection, process using viral particles and other known methods (See "Genetic Engineering Handbook", Extra edition of "Experimental Medicine", issued on March 20, 1991, Yodo-sha), and any method may be employed. The transformant of the present invention may be used for the purpose of producing the DNA according to the second aspect of the present invention in a large amount. In addition, when the DNA according to the second aspect of the present invention is incorporated in the downstream of an adequate promoter in the host cell, the transformant will produce the fusion protein according to the first aspect of the present invention. Therefore, such transformant can be used, for example, for the purpose of producing the fusion protein according to the first aspect of the present invention. The transformant according to the fourth aspect of the present invention may be either a prokaryotic cell or a eukaryotic cell. Typical prokaryotic cells include Escherichia coli and Bacillus subtilis. Typical eukaryotic cells include CHO cell, HeLa cell, COS cell, Namalwa cell, and other mammal cells as well as Sf cell or other insect cell and yeast. The transformant is preferably the one which produces the fusion protein of the present invention, and more preferably, the one which secretes the fusion protein of the present invention in the culture. One most preferable example is COS-1 cell.

Next, the method for increasing the production of the recombinant protein according to the fifth aspect of the present invention is described, and this method is characterized in that the desired protein is produced as a fusion protein with FLAG-like peptide. Increase in the production of the recombinant protein means, for example, increase in the amount of the fusion protein recovered from the culture supernatant in the production of fusion protein by a mammal cell, or increase in the amount of fusion protein recoverable from the lysate of Escherichia coli in the production of the fusion protein using Escherichia coli.

The characteristic feature of the present method is that the FLAG-like peptide is not used merely as a purification tag as in the case of conventional methods but as a sequence for increasing the production of the recombinant protein. A known sequence which increases the production of the recombinant protein is the secretion signal sequence which facilitates secretion of the protein from the cell. While FLAG-like peptide is not a secretion signal sequence, the inventor of the present invention newly found that the FLAG-like peptide has the effect of increasing the production of the recombinant protein. As shown in Example 2, in the production of the extracellular domain of Fas ligand in COS-1 cell, the amount produced became increased by about 3 folds when FLAG peptide was ligated to the N terminal side of the extracellular domain of the Fas ligand. On the other hand, when leucine zipper was ligated to the N terminal side of the extracellular domain of the Fas ligand for the purpose of increasing the biological activity, the amount expressed drastically became reduced by the presence of the leucine zipper. However, when FLAG peptide was additionally ligated, the amount produced increased by 20 to 35 folds. There is a problem that the amount produced drastically reduces when a protein such as a cytokine wherein formation of an oligomer is important for its activity is produced as a fusion protein with a peptide having oligomerization ability, and in such a case, the production can be increased by additionally fusing the FLAG-like peptide. The method for producing the desired protein as a fusion protein with the FLAG-like peptide will be described with regard to the sixth aspect of the present invention.

Next, the method for producing a recombinant protein according to the sixth aspect of the present invention is described, and this method comprises the steps of producing an expression vector including a DNA fragment comprising the nucleotide sequence coding for the desired protein ligated to the nucleotide sequence coding for FLAG-like peptide with their reading frame matched, introducing said expression vector in an adequate host cell or microorganism, cultivating the resulting transformant in the condition suitable for expression, and recovering the recombinant protein from the culture mixture and purifying the recovered recombinant protein, whereby production of the desired protein is increased.

The order of ligating the nucleotide sequence coding for the desired protein and the nucleotide sequence coding for the FLAG-like peptide in the DNA fragment is not limited as long as the effect of increasing the protein production by the FLAG-like peptide is attained, and the DNA fragment may further include the nucleotide sequence coding for an arbitrary linker sequence or signal sequence. The expression vector has been described with regard to the third aspect of the present invention, and the transformant has been described with regard to the fourth aspect of the present invention.

The transformant may be cultivated by any method generally employed in the art, which may be carried out by referring to various books (for example, "Experimental Methods in Microbiology" edited by incorporated association of Japanese Society of Biochemistry and published by Tokyo Kagaku Dojin K.K. in 1992). The method employed and the need for amplifying the gene or inducing the expression may vary depending on the type of the host cell and the promoter used. The expression, for example, may be accomplished by using 3β-indole acrylate when the promoter used is trp promoter, by dexamethazone when the promoter used is MMTV promoter, and by methanol when the promoter used is AOX1 promoter. The gene may be amplified with methotrexate when an expression vector containing DHFR (dihydrofolate reductase) gene is used.

In the sixth aspect of the present invention, the "culture mixture" designates either the supernatant or the cell. To be more specific, when the transformant secretes the recombinant protein to the exterior of the cell, the protein may be recovered and purified from the supernatant. On the other hand, when the recombinant protein is accumulated in the host cell, the protein may be recovered by lysing the cell using a lysozyme, a surfactant, freeze thawing, pressure application, or the like, subjecting the lysate to centrifugation to recover the supernatant, removing unnecessary cell debris and the like from the supernatant by filtration and the like, and purifying the recombinant protein from the thus treated supernatant. When the transformant used is Escherichia coli, and the protein produced is accumulated in the periplasm, such process may be accomplished by the method of Wilsky et al. (J.Bacteriol. 127: 595, 1976). The recombinant protein may be purified from the culture mixture by any of the methods generally used in the art in purifying the protein. To be more specific, the recombinant protein may be purified by conducing an adequate method selected from salt precipitation, ultrafiltration, isoelectric precipitation, gel filtration, electrophoresis, ion change chromatography, hydrophobic chromatography, antibody chromatography or other affinity chromatography, chromatofocusing, absorption chromatography, reversed phase chromatography, and the like in an arbitrary order, and if desired, by also using HPLC system. A preferable example of such method is the one shown in Example 4. Also preferred is the affinity chromatography using an antibody specific to the FLAG-like peptide as disclosed in USP 5,011,912. The FLAG-like peptide comprising the amino acid sequence of Asp-Asp-Asp-Asp-Lys may be cleaved by using an enterokinase, and the recombinant protein can be obtained as a protein from which the FLAG-like peptide has been removed. The "increase in the production of the protein" in regard of the fifth and sixth aspects of the present invention means that the amount produced has increased by at least 1.5 folds, preferably by at least 2 folds, more preferably by at least 3 folds, still more preferably by at least 20 folds, and most preferably by at least 30 folds compared to the case when the protein is not produced as a fusion protein with the FLAG-like peptide. The amount of the protein produced may be evaluated by the EIA system as shown in Example 2 using the antibody specific to the target protein, or by the method generally used in the art.

Next, the method for increasing the biological activity of a fusion protein of leucine zipper and a transmembrane protein according to the seventh aspect of the present invention is described. The fusion protein has FLAG-like peptide ligated thereto in the process of producing said fusion protein, and the biological activity as a transmembrane protein of the fusion protein having the FLAG-like peptide ligated thereto is thereby increased. Leucine zipper has been described with regard to the first aspect of the present invention. Examples of the transmembrane protein include TNFα, Fas ligand, TRAIL, CD40 ligand and other members of the TNF family, TNF receptor, Fas, DR4 and other members of the TNF/NGF receptor family, as well as cytokine receptors of hemopoietin receptor family and interferon receptor family. The "biological activity" of such transmembrane protein means, at least, the biological activity to bind to the corresponding ligand in the case when the transmembrane protein is a receptor, and the biological activity to bind to the corresponding receptor in the case when the transmembrane protein is a ligand. A transmembrane protein generally has the function of transducing a signal to the interior of the cell. However, as long as the transmembrane protein has its binding activity retained, the transmembrane protein may not necessarily have the activity of transducing the signal to the interior of the cell upon binding of the transmembrane protein with the receptor/ligand. When the transmembrane protein has the activity of binding with the receptor/ligand without transducing the signal, the transmembrane protein can be used to suppress the change in the cell phenotype induced by the signal. However, the transmembrane protein is preferably the one having the activity of transducing the signal to the interior of the cell upon binding of the transmembrane protein with the receptor/ligand to induce the change in the cell phenotype.

The fusion protein produced by the method according to the seventh aspect of the present invention is constituted from the FLAG-like peptide, the leucine zipper, and the transmembrane protein, and as long as the biological activity of the transmembrane protein is retained, the three types peptide may be ligated in any order and the fusion protein may further include any type of linker sequence or signal sequence. However, as long as the transmembrane protein retains its biological activity, the transmembrane protein may be a peptide comprising the full length of the amino acid sequence; a peptide which is defined by the amino acid sequence comprising an arbitrary part of the amino acid sequence; a peptide which is defined by the amino acid sequence comprising an arbitrary part of the amino acid sequence and at least one amino acid of any type such as Met attached to either or both of the N and C terminals of said part; or a peptide which comprises an amino acid sequence wherein one to several amino acids have been mutated, deleted, substituted, or added in the amino acid sequence. The extracellular domain of the transmembrane protein is surely a typical such peptide.

A typical case where application of the method according to the seventh aspect of the present invention may bring favorable results is the case wherein the extracellular domain of Fas ligand is used for the transmembrane protein. As shown in Example 3, the fusion protein wherein peptides were ligated in the order of FLAG peptide - leucine zipper - human Fas ligand extracellular domain exhibited an apoptosis-inducing activity which was about 10 folds higher than the fusion protein having no FLAG peptide ligated thereto, namely, the fusion protein of leucine zipper - human Fas ligand extracellular domain. In other words, we succeeded in increasing the Fas ligand biological activity of the fusion protein of the leucine zipper and the Fas ligand by additionally ligating the FLAG peptide. The "increase in the biological activity" used in regard of the seventh aspect of the present invention means that at least one activity value is at least higher than the case when the protein is not produced as a fusion protein with the FLAG-like peptide, for example, that the activity value is higher by at least 1.5 folds, preferably by at least 2 folds, more preferably by at least 3 folds, still more preferably by at least 5 folds, and most preferably by at least 10 folds. Comparison of the activity value and the like may be accomplished by the methods generally used in the art, and most typically, by comparing the activity per unit substance, namely, by comparing the specific activity. The specific activity is typically determined in terms of 50% inhibitory concentration ($IC_{50}$) or 50% effective dose ($ED_{50}$).

EXAMPLES

Next, the present invention is described in further detail by referring to the illustrative Examples which by no means limit the scope of the present invention. The abbreviations used in the following description are based on those used in the art.

(Example 1) Construction of plasmid vector expressing human Fas ligand fusion protein

(1) Plasmid pM1807 which expresses fusion protein of leucine zipper and the extracellular domain of human Fas ligand was produced by the procedure as described below.

Sense primer 1 (ACCATGCTGGGCATCTGGACCCTCCTACCTCTGGTTCTTACGTCTGTTGCT), antisense primer 1 (ATTTCTTCGATCTTGTCTTCGATTTGTTTCATTCTAGCAACAGACGTAAGAACCAG), sense primer 2 (GAAGACAAGATCGAAGAAATTCTTTCGAAAATCTATCACATCGAAAATGAG), antisense primer 2 (GCGTTCGCCGATTAATTTCTTGATTCTGGCAATCTCATTTTCGATGTGATAGA), sense primer 3 (TGCGAATTCACCATGCTGGGCATCTGG), antisense primer 3 (GGAAGAGCTGCAGCAGGCGTTCGCCGATTAATTTC), sense primer 4 (GGCGAACGCCTGCTGCAGCTCTTCCACCTACAG), and antisense primer 4 (AATAAGCTTGGTACCCTATTAGAGCTTATATAA) were synthesized by a chemical synthesizer. Sense primer 1 includes the sequence coding for the human Fas signal sequence. Antisense primer 1 includes the complementary sequence to 3' terminal region of the human Fas signal sequence and 5' terminal region of the isoleucine zipper. Sense primer 2 includes intermediate region of the sequence coding for the leucine zipper. Antisense primer 2 includes the complementary sequence to 3' terminal region of the leucine zipper. Sense primer 3 includes 5' terminal region of the sequence coding for the human Fas signal sequence, and EcoRI site (GAATTC). Antisense primer 3 includes the complementary sequence to 3' terminal region of leucine zipper, PstI site (CTGCAG), and N terminal side of the extracellular domain of human Fas ligand. Sense primer 4 includes 3' terminal region of the sequence coding for leucine zipper, PstI site (CTGCAG), and the nucleotide sequence coding for N terminal side of the extracellular domain of human Fas ligand. Antisense primer 4 includes the complementary sequence to C terminal side of the human Fas ligand, TAA termination codon, and KpnI site (GGTACC).

A 50 μL solution containing 50 pmol of each of the sense primer 1 and antisense primer 1, or sense primer 2 and antisense primer 2, 10 nmol of each of dATP, dCTP, dGTP, and dTTP, 1.25 units of Pfu DNA polymerase (Stratagene), and 5 μL of the 10 x Pfu buffer was prepared. PCR was performed by repeating cycle 30 times consisting of 30 seconds at 94°C, 30 seconds at 55°C, and 1 minute at 72°C by using DNA Thermal Cycler (PCR system 9600, PE Biosystems). A 50 μL PCR reaction mixture containing 0.5 μL of each of the PCR product, and 50 pmol of each of sense primer 3 and antisense primer 3 was prepared, and PCR was performed in the manner as described above.

In the meantime, a 50 μL PCR reaction mixture containing 50 pmol of each of sense primer 4 and antisense primer 4 and 1 ng of plasmid pBX-hFL1 (WO 95/13293) including the sequence coding for human Fas ligand as a template was prepared, and PCR was performed. In addition, a 50 μL PCR reaction mixture containing 0.5 μL of each of the PCR product obtained by using sense primer 3 and antisense primer 3, and the PCR product obtained by using sense primer 4 and antisense primer 4, and 50 pmol of each of sense primer 3 and antisense primer 4 was prepared, and PCR was performed.

The resulting PCR product was double digested with EcoRI and KpnI. In the meanwhile, expression plasmid pM1070 (WO 95/13293) coding for the extracellular domain of human Fas ligand following EF promoter and comprising DHFR gene was double digested with EcoRI and KpnI, and the fragment having the size of about 7kbp was recovered and purified by agarose gel electrophoresis. The fragment obtained from the plasmid was ligated to the fragment of the PCR product that had been digested with EcoRI and KpnI, and the resulting plasmid was designated pM1807.

(2) Plasmid pM1809 which expresses fusion protein of FLAG peptide and the extracellular domain of human Fas ligand was produced by the procedure as described below.

Antisense primer 5 (CTTGTCATCGTCATCCTTGTAGTCAGCAACAGACGTAAGAACC) and sense primer 5 (GACTACAAGGATGACGATGACAAGCAGCTCTTCCACCTACAG) were synthesized by a chemical synthesizer. This antisense primer 5 includes the complementary sequence to 3' terminal region of human Fas signal sequence and FLAG peptide. Sense primer 5 includes the sequence coding for FLAG peptide and the nucleotide sequence coding for N terminal side of the extracellular domain of human Fas ligand.

A 50 µL PCR reaction mixture containing 50 pmol of each of the thus obtained antisense primer 5 and sense primer 3 produced in Example 1(1), and 0.05 pmol of sense primer 1 as the template was prepared, and PCR was performed as described in Example 1(1). In the meanwhile, a 50 µL PCR reaction mixture containing 50 pmol of each of sense primer 5 and antisense primer 4 produced in Example 1(1), and 1 ng of pBX-hFL1 used in Example 1(1) as the template was prepared, and PCR was performed. A 50 µL PCR reaction mixture containing 0.5 µL of each of the PCR product, and 50 pmol of each of sense primer 3 and antisense primer 4 was prepared, and PCR was performed.

The thus obtained PCR product was double digested with EcoRI and KpnI, and as in the case of Example 1 (1), the digestion product was ligated to the fragment obtained from the plasmid pM1070 which had been double digested with EcoRI and KpnI. The resulting plasmid was designated pM1809.

(3) Plasmid pUC-IZFL was produced by the procedure as described below. pUC118 (Takara Shuzo Co.) was digested with PstI, blunted using DNA Blunting Kit (Takara Shuzo Co.) and self-ligated, so the plasmid pUC118 was deleted the PstI site. This plasmid was double digested with EcoRI and KpnI, and this plasmid was ligated to the EcoRI-KpnI double digested fragment of the PCR product including leucine zipper and the extracellular domain of human Fas ligand produced in Example 1(1). The resulting plasmid was designated pUC-IZFL.

(4) Plasmid pM1815 which expresses fusion protein (SEQ ID NO:4) of FLAG peptide, leucine zipper and the extracellular domain of human Fas ligand was produced by the procedure as described below.

Antisense primer 6 (GTTTCATTCTCTTGTCATCGTCATCCTTGTA) and sense primer 6 (CGATGACAAGAGAATGAAACAAATCGAAGAC) were synthesized in a chemical synthesizer. This antisense primer 6 includes the complementary sequence to the sequence coding for FLAG peptide and 5' terminal region of leucine zipper. Sense primer 6 includes 3' terminal region of FLAG peptide and 5' terminal region of leucine zipper.

A 50 µL PCR reaction mixture containing 50 pmol of each of the antisense primer 6 and sense primer 3 produced in Example 1(1), and 1 ng of pM1809 produced in Example 1(2) as the template was prepared, and PCR was performed as described in Example 1(1). In the meanwhile, a 50 µL PCR reaction mixture containing 50 pmol of each of sense primer 6 and antisense primer 3 produced in Example 1(1), and 1 ng of pM1807 produced in Example 1(1) as the template was prepared, and PCR was performed. A 50 µL PCR reaction mixture containing 0.5 µL of each of the PCR product, and 50 pmol of each of sense primer 3 and antisense primer 3 was prepared, and PCR was performed.

The thus obtained PCR product was double digested with EcoRI and PstI. In the meanwhile pUC-IZFL produced in Example 1(3) was double digested with EcoRI and PstI, and the fragment of about 3.7kbp was recovered and purified by agarose gel electrophoresis. This fragment obtained from the plasmid was ligated to the fragment of the PCR product that had been double digested with EcoRI and PstI as described above. This constructed plasmid was further double digested with EcoRI and KpnI, and as in the case of Example 1(1), the digested fragment was ligated to the fragment obtained from the plasmid pM1807 that had been double digested with EcoRI and KpnI. The resulting plasmid was designated pM1815. The inventor of the present invention deposited plasmid pM1815 to the National Institute of Advanced Industrial Science and Technology (Independent Administrative Institute), International Patent Organism Depositary (1-3, Higashi 1-chome, Tsukuba-shi, Ibaraki-ken, Japan) on May 12, 2000 (Accession No. FERM P-17853), and it was transferred from the original deposition to the international deposition on May 8, 2001 (Accession No. FERM BP-7575).

(Example 2) Expression of human Fas ligand fusion protein

(1) Human Fas ligand fusion protein was expressed using COS-1 cell by the procedure as described below. COS-1 cell was transfected with pM1815, pM1809, pM1807 or pM1070 (WO 95/13293) produced in Example 1 and expressed the protein in the supernatant. To be more specific, 1 μg of plasmid was dissolved in 2 μL of 10mM Tris-HCl (pH7.4)/1mM ethylenediamine tetraacetate solution. This plasmid solution was added to 0.7 mL of D-MEM (Nissui Pharmaceutical Co., Ltd) containing 0.2 mg/mL DEAE-dextran and 50mM Tris-HCl (pH7.4) to prepare DNA-DEAE dextran mixed solution. The DNA-DEAE dextran mixed solution was added dropwise to COS-1 cell which had been cultivated to semiconfluency in a 6 well plate, and the cells were cultivated at 37°C in a $CO_2$ incubator. After 4 hours, the DNA-DEAE dextran mixed solution was removed and replaced with D-MEM containing 10% FBS (Gibco). Cultivation was continued for another 96 hours. The supernatant of the COS-1 cell having the plasmid introduced therein was recovered for use in the following (2) and Example 3.

(2) The human Fas ligand fusion protein in the COS-1 cell supernatant was quantitated by the procedure as described below. The human Fas ligand fusion protein in the supernatant was quantitated by means of EIA (Enzyme immuno assay) using antibodies specific to Fas ligand (F918-20-2 as the immobilized antibody, and F919-9-18 as the horseradish peroxidase-labeled antibody; see WO 97/02290 for the detail of the antibody) (Bone Marrow Transplantation 22: 751, 1998). As shown in Table 1, compared to the extracellular domain of human Fas ligand (shFasL) expressed by pM1070, the fusion protein FLAG-shFasL expressed by pM1809, additionally including the FLAG peptide fused thereto, was produced at a larger amount by about 3 folds. Furthermore, while IleZip - shFasL (pM1807) including leucine zipper was produced at a drastically reduced amount, FLAG - IleZip - shFasL (pM1815) additionally including FLAG peptide fused thereto was expressed at an amount about 30 folds larger than that of the IleZip - shFasL.

Table 1:

| Effects of FLAG peptide on the production of Fas ligand fusion proteins | | |
|---|---|---|
| | Exp.#1 | Exp.#2 |
| shFasL (pM1070) | 448(ng/mL) | 379(ng/mL) |
| FLAG-shFasL (pM1809) | 1410(ng/mL) | 1360(ng/mL) |
| IleZip-shFasL (pM1807) | 15.5(ng/mL) | 17.8(ng/mL) |
| FLAG-IleZip-shFasL (pM1815) | 542(ng/mL) | 362(ng/mL) |

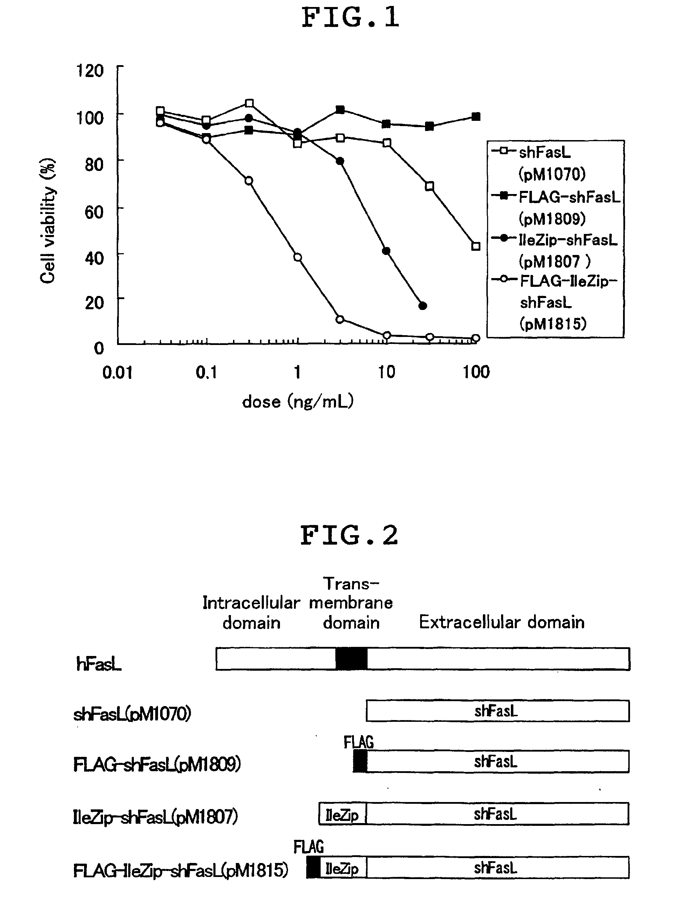

(Example 3) The Fas ligand fusion proteins in the cell supernatant were evaluated for their apoptosis-inducing activity by the WST-1 assay as described below. Jurkat cell which is a cell line from human T cell was suspended in RPMI1640 medium (Nissui Pharmaceutical K.K.) that had been supplemented with 10% FBS at $4 \times 10^5$ cells/mL, and the suspended cells were inoculated into the wells of a 96 well plate at 50 μL/well ($2 \times 10^4$ cells/well). Next, the COS-1 cell supernatant containing the Fas ligand fusion protein produced in Example 2 was diluted with RPMI1640 medium supplemented with 10% FBS to the assay concentration. This solution was added at 50 μL/well to the well that had been inoculated with the cell, and after the cultivating at 37°C in a CO2 incubator for about 20 hours, the apoptosis-inducing activity was evaluated. The evaluation was conducted by using WST-1 reagent (Premix WST-1 Cell Proliferation Assay System, Takara Shuzo Co.), which assays mitochondria enzymatic activity of the living cells. The reagent was added at 10 μL /well, and after incubating at 37°C in a $CO_2$ incubator for 0.5 to 2 hours, absorption (450 nm - 620 nm) was measured. To conducted the calculation of the apoptosis-inducing activity, the absorbance of the well in which the cells had not been inoculated, was subtracted from that of each sample well so as to remove background, and the apoptosis-inducing activity of each sample was represented by percentage to the value of the control well which is free from Fas ligand fusion protein. The results are shown in FIG. 1.

```
Cell viability (%) =
```

$$\frac{(\text{absorption of the well being measured}) - (\text{absorption of the well including no cell})}{(\text{absorption of the well with no addition of Fas ligand fusion protein}) - (\text{absorption of the well including no cell})} \times 100$$

As shown in FIG. 1, FLAG - IleZip - shFasL exhibited dose dependent cytotoxicity to the Jurkat cells. Furthermore, the apoptosis-inducing activity of the FLAG-IleZip - shFasL was about 10 times higher than that of the IleZip - shFasL including no FLAG peptide fused thereto, and about 100 times higher than that of the shFasL including no leucine zipper and FLAG peptide fused thereto. In addition, these apoptosis-inducing activities were completely suppressed by F919-9-18 which is an anti-human Fas ligand neutralizing antibody (see WO 097/02290 for the detail), demonstrating that such apoptosis-inducing activity was specific to the Fas-Fas ligand system.

(Example 4) Purification of human Fas ligand fusion protein

The supernatant of COS-1 cell containing FLAG - IleZip - shFasL prepared by the procedure of Example 2 (1) was purified as described below by means of affinity chromatography using Sepharose 4B immobilizing anti-Fas ligand antibody F919-9-18 (described in WO 097/02290). 29,420 mL of the COS-1 cell supernatant containing FLAG-IleZip - shFasL was passed through a filter having a pore size of 0.45 μm (Millipak 60: Millipore), and the filtrate was recovered for use as a material in further purification. This purification material was applied to F919-9-18-Sepharose 4B FF column (3.2 cm (diam) $\times$ 6.2 cm) which had been preliminarily equilibrated with phosphate-buffered saline(PBS-) at a flow rate of 10 mL/min in a cold place. After the application of the material, the column was washed by applying PBS- at 15.3 mL/min (washing 1), and then, 1 mol/L NaCl/PBS- under the same condition (washing 2). The column was then applied with 50 mmol/L glycine-NaOH (pH 11) at 10 mL/min for elution. 10 mL of 1 mol/L Tris-HCl (pH 8) was quickly added per 40 mL of the eluted fraction, and the eluate was stored in cool place. Amount of the FLAG - IleZip - shFasL in each fraction was measured by the procedure described in Example 2, and the fraction containing the FLAG - IleZip - shFasL was collected.
The table of the purification is shown in Table 2.

Table 2

| F919-9-18-Sepharose 4B FF column chromatography | | | | |
|---|---|---|---|---|
| Sample | Amount of liquid (mL) | FLAG-IleZip-shFasL (ng/mL) | Total amount of FLAG-IleZip-shFasL (mg) | Recovery rate (%) |
| Culture supernatant | 29,420 | 1,390 | 40.8 | 100 |
| Starting filtrate | 30,210 | 1,260 | 38.2 | 93.6 |
| Unadsorbed + washing 1 | 33,000 | 1.58 | 0.05 | 0.1 |
| Washing 2 | 290 | 0.00 | 0.00 | 0.0 |
| Fraction 1 | 47.9 | 0.00 | 0.00 | 0.0 |
| Fraction 2 | 138.6 | 247,000 | 34.2 | 83.8 |
| Fraction 3 | 243.6 | 645 | 0.16 | 0.4 |

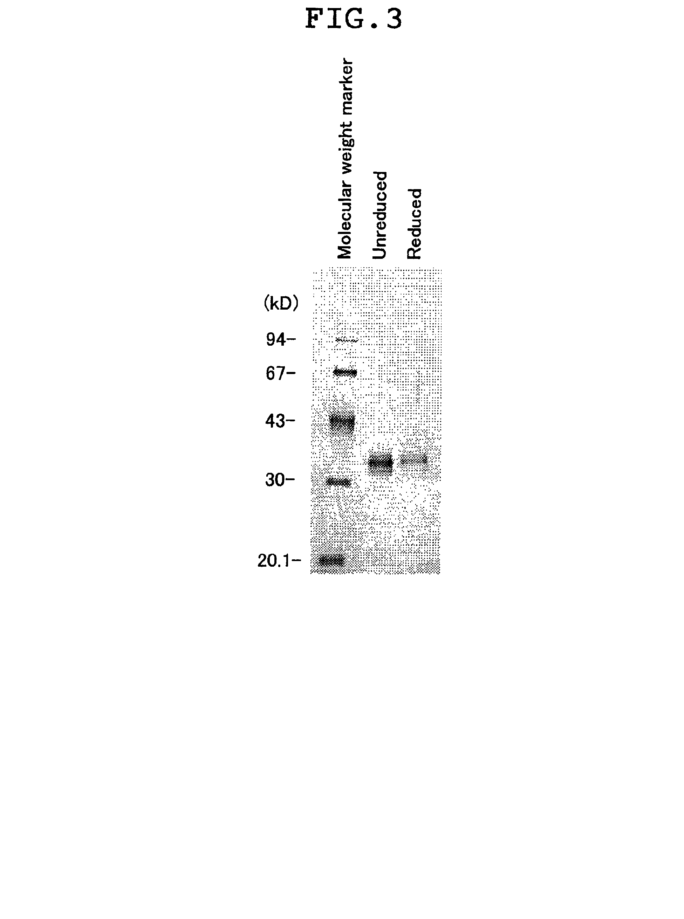

As shown in Table 2, FLAG - IleZip - shFasL was found only in the eluted fractions. Purified FLAG - IleZip-shFasL was recovered by F919-9-18-Sepharose 4B FF column chromatography at a recovery rate of 84%. The purified product was electrophoresed by SDS-PAGE, followed by silver-staining (2D-Silver Stain II (first): Daiichi Pure Chemicals Co., Ltd.), in which the product was detected as a single band (FIG. 3). The cytotoxicity of the purified product was confirmed by the method described in Example 3. It was then found that the purified product had an activity equivalent to that of the supernatant before the purification.

INDUSTRIAL APPLICABILITY OF THE INVENTION

According to the present invention, there is provided a fusion protein which has Fas ligand biological activity. The Fas ligand fusion protein provided by the present invention is the fusion protein which had been fused with a peptide having oligomerization ability and a peptide capable of increasing the production of a recombinant protein and increasing the biological activity of the fusion protein of the leucine zipper and the transmembrane protein, and as a consequence, high biological activity as well as high production are realized. This Fas ligand fusion protein can be developed into a therapeutic agent for diseases wherein Fas-mediated apoptosis is involved. This invention also provides a method for increasing the production of a recombinant protein which is useful in the production of the above-described fusion protein and which can be generally applied in the production of the recombinant protein, and a method for increasing the biological activity of the fusion protein of the leucine zipper and the transmembrane protein.

**Claims**

1. A fusion protein which is capable of binding to Fas, and which comprises (a) a peptide comprising at least a part of the amino acid sequence of Fas ligand, (b) a peptide having oligomerization ability, and (c) a peptide which increases recombinant protein production.

2. A fusion protein according to claim 1 wherein
   said peptide (a) comprising at least a part of the amino acid sequence of Fas ligand is selected from the group consisting of (a-1) a peptide comprising the amino acid sequence of SEQ ID NO:1, (a-2) a peptide comprising amino acids 103 to 281 of the amino acid sequence of SEQ ID NO:1, (a-3) a peptide comprising amino acids 130 to 281 of the amino acid sequence of SEQ ID NO:1, (a-4) a peptide at least comprising 145 to 281 amino acids of the amino acid sequence of SEQ ID NO:1, and (a-5) a peptide having Fas-binding activity which comprises an amino acid sequence wherein one to several amino acids have been deleted, substituted, or added in the amino acid sequence of any one of (a-1) to (a-4);
   said peptide (b) having oligomerization ability is selected from the group consisting of (b-1) leucine zipper, (b-2) a peptide comprising the amino acid sequence of SEQ ID NO:2, and (b-3) a peptide having oligomerization ability which comprises an amino acid sequence wherein one to several amino acids have been deleted, substituted, or added in the amino acid sequence of SEQ ID NO:2; and
   said peptide (c) which increases recombinant protein production is selected from the group consisting of (c-1) FLAG-like peptide, (c-2) a peptide comprising an amino acid sequence of Asp-B-Z-Asp-Asp-Asp-Asp-Lys (wherein B-Z is Tyr-Lys or Leu-Tyr), and (c-3) a peptide comprising an amino acid sequence of Asp-Tyr-Lys-$X_{1-n}$-R (wherein R is Lys, Arg, Met, or Asn; and $X_{1-n}$ represents an amino acid other than Lys, Arg, Met, and Asn).

3. A fusion protein according to claim 2 **characterized in that** said fusion protein has an activity of inducing apoptosis in a Fas-expressing cell.

4. A fusion protein according to claim 3 **characterized in that** said activity of inducing apoptosis in a Fas-expressing cell is such that cell viability in WST-1 assay upon addition said fusion protein at an amount of 3 ng/mL is 50% or less.

5. A fusion protein according to any one of claims 2 or 4 wherein said peptide (c), said peptide (b), and said peptide (a) are connected in this order from the N terminal side.

6. A fusion protein according to any one of claims 2 to 5 further comprising a signal sequence.

7. A protein which is either one of the following (d) and (e) :
   (d) a protein comprising the amino acid sequence of SEQ ID NO:4;
   (e) a protein having Fas-binding activity which comprising an amino acid sequence wherein one to several amino acids have been deleted, substituted, or added in the amino acid sequence of SEQ ID NO:4.

8. A DNA coding for the fusion protein according to any one of claims 1 to 7.

9. An expression vector containing the DNA of claim 8.

10. A transformant produced by transformation using the expression vector of claim 9.

11. A method for producing a recombinant protein **characterized in that** a desired protein is produced in the form of a protein fused with FLAG-like peptide to thereby increase the amount of said desired protein produced.

12. A method according to claim 11 wherein said desired protein is a transmembrane protein.

13. A method according to claim 11 wherein said desired protein is the extracellular domain of a transmembrane protein.

14. A method according to claim 11 wherein said desired protein is a fusion protein of a peptide having oligomerization ability and a transmembrane protein.

15. A method according to claim 11 wherein said desired protein is a fusion protein of a peptide having oligomerization ability and the extracellular domain of a transmembrane protein.

**16.** A method according to claim 14 or 15 wherein said peptide having oligomerization ability is leucine zipper, and said transmembrane protein is Fas ligand.

**17.** A method for producing a recombinant protein **characterized in that** said method comprises the steps of producing an expression vector including a DNA fragment comprising the nucleotide sequence coding for the desired protein ligated to the nucleotide sequence coding for FLAG-like peptide with their reading frame matched; introducing said expression vector in a host; cultivating the resulting transformant in the condition suitable for expression; and recovering the recombinant protein from the culture mixture and purifying the recovered recombinant protein to thereby increase production of said desired protein.

**18.** A method for increasing the biological activity of a fusion protein of leucine zipper and a transmembrane protein wherein FLAG-like peptide is further ligated to the fusion protein in the process of producing said fusion protein.

**19.** A method for increasing the biological activity of a fusion protein of leucine zipper and the extracellular domain of a transmembrane protein wherein FLAG-like peptide is further ligated to the fusion protein in the process of producing said fusion protein.

# FIG.1

# FIG.2

# FIG.3

Sequence Listing

<110> Mochida Pharmaceutical Co., LTD.

<120> Fas Ligand Fusion Protein

<130> MD0582

<160> 4

<170> PatentIn Ver. 2.1

<210> 1

<211> 281

<212> PRT

<213> human

<400> 1

Met Gln Gln Pro Phe Asn Tyr Pro Tyr Pro Gln Ile Tyr Trp Val
                    5                   10                  15

Asp Ser Ser Ala Ser Ser Pro Trp Ala Pro Pro Gly Thr Val Leu
                    20                  25                  30

Pro Cys Pro Thr Ser Val Pro Arg Arg Pro Gly Gln Arg Arg Pro
                    35                  40                  45

Pro Pro Pro Pro Pro Pro Pro Pro Leu Pro Pro Pro Pro Pro Pro
                    50                  55                  60

Pro Pro Leu Pro Pro Leu Pro Leu Pro Pro Leu Lys Lys Arg Gly

                    65                    70                    75

Asn His Ser Thr Gly Leu Cys Leu Leu Val Met Phe Phe Met Val

                    80                    85                    90

Leu Val Ala Leu Val Gly Leu Gly Leu Gly Met Phe Gln Leu Phe

                    95                    100                   105

His Leu Gln Lys Glu Leu Ala Glu Leu Arg Glu Ser Thr Ser Gln

                    110                   115                   120

Met His Thr Ala Ser Ser Leu Glu Lys Gln Ile Gly His Pro Ser

                    125                   130                   135

Pro Pro Pro Glu Lys Lys Glu Leu Arg Lys Val Ala His Leu Thr

                    140                   145                   150

Gly Lys Ser Asn Ser Arg Ser Met Pro Leu Glu Trp Glu Asp Thr

                    155                   160                   165

Tyr Gly Ile Val Leu Leu Ser Gly Val Lys Tyr Lys Lys Gly Gly

                    170                   175                   180

Leu Val Ile Asn Glu Thr Gly Leu Tyr Phe Val Tyr Ser Lys Val

                    185                   190                   195

Tyr Phe Arg Gly Gln Ser Cys Asn Asn Leu Pro Leu Ser His Lys

                    200                   205                   210

Val Tyr Met Arg Asn Ser Lys Tyr Pro Gln Asp Leu Val Met Met

                    215                   220                   225

Glu Gly Lys Met Met Ser Tyr Cys Thr Thr Gly Gln Met Trp Ala

```
              230                 235                 240

Arg Ser Ser Tyr Leu Gly Ala Val Phe Asn Leu Thr Ser Ala Asp

              245                 250                 255

His Leu Tyr Val Asn Val Ser Glu Leu Ser Leu Val Asn Phe Glu

              260                 265                 270

Glu Ser Gln Thr Phe Phe Gly Leu Tyr Lys Leu

              275                 280 281
```

<210> 2

<211> 33

<212> PRT

<223> artificial

<400> 2

```
Arg Met Lys Gln Ile Glu Asp Lys Ile Glu Glu Ile Leu Ser Lys

                 5                 10                  15

Ile Tyr His Ile Glu Asn Glu Ile Ala Arg Ile Lys Lys Leu Ile

                20                 25                  30

Gly Glu Arg

       33
```

<210> 3

<211> 8

<212> PRT

<213> artificial

<400> 3

Asp Tyr Lys Asp Asp Asp Asp Lys


<210> 4

<211> 714

<212> DNA

<213> artificial

<400> 4

atg ctg ggc atc tgg acc ctc cta cct ctg gtt ctt acg tct gtt gct

Met Leu Gly Ile Trp Thr Leu Leu Pro Leu Val Leu Thr Ser Val Ala

1               5                   10                  15

gac tac aag gat gac gat gac aag aga atg aaa caa atc gaa gac aag

Asp Tyr Lys Asp Asp Asp Asp Lys Arg Met Lys Gln Ile Glu Asp Lys

                20                  25                  30

atc gaa gaa att ctt tcg aaa atc tat cac atc gaa aat gag att gcc

Ile Glu Glu Ile Leu Ser Lys Ile Tyr His Ile Glu Asn Glu Ile Ala

            35                  40                  45

aga atc aag aaa tta atc ggc gaa cgc ctg ctg cag ctc ttc cac cta

Arg Ile Lys Lys Leu Ile Gly Glu Arg Leu Leu Gln Leu Phe His Leu

        50                  55                  60

```
cag aag gag ctg gca gaa ctc cga gag tct acc agc cag atg cac aca
Gln Lys Glu Leu Ala Glu Leu Arg Glu Ser Thr Ser Gln Met His Thr
                                                                80
65              70              75
```

```
gca tca tct ttg gag aag caa ata ggc cac ccc agt cca ccc cct gaa
Ala Ser Ser Leu Glu Lys Gln Ile Gly His Pro Ser Pro Pro Pro Glu
                85              90              95
```

```
aaa aag gag ctg agg aaa gtg gcc cat tta aca ggc aag tcc aac tca
Lys Lys Glu Leu Arg Lys Val Ala His Leu Thr Gly Lys Ser Asn Ser
                100             105             110
```

```
agg tcc atg cct ctg gaa tgg gaa gac acc tat gga att gtc ctg ctt
Arg Ser Met Pro Leu Glu Trp Glu Asp Thr Tyr Gly Ile Val Leu Leu
                115             120             125
```

```
tct gga gtg aag tat aag aag ggt ggc ctt gtg atc aat gaa act ggg
Ser Gly Val Lys Tyr Lys Lys Gly Gly Leu Val Ile Asn Glu Thr Gly
            130             135             140
```

```
ctg tac ttt gta tat tcc aaa gta tac ttc cgg ggt caa tct tgc aac
Leu Tyr Phe Val Tyr Ser Lys Val Tyr Phe Arg Gly Gln Ser Cys Asn
145             150             155             160
```

```
aac ctg ccc ctg agc cac aag gtc tac atg agg aac tct aag tat ccc
Asn Leu Pro Leu Ser His Lys Val Tyr Met Arg Asn Ser Lys Tyr Pro
                165             170             175
```

```
cag gat ctg gtg atg atg gag ggg aag atg atg agc tac tgc act act
```

Gln Asp Leu Val Met Met Glu Gly Lys Met Met Ser Tyr Cys Thr Thr

       180             185             190

ggg cag atg tgg gcc cgc agc agc tac ctg ggg gca gtg ttc aat ctt

Gly Gln Met Trp Ala Arg Ser Ser Tyr Leu Gly Ala Val Phe Asn Leu

       195             200             205

acc agt gct gat cat tta tat gtc aac gta tct gag ctc tct ctg gtc

Thr Ser Ala Asp His Leu Tyr Val Asn Val Ser Glu Leu Ser Leu Val

       210             215             220

aat ttt gag gaa tct cag acg ttt ttc ggc tta tat aag ctc

Asn Phe Glu Glu Ser Gln Thr Phe Phe Gly Leu Tyr Lys Leu

225            230            235